# EUROPEAN PATENT APPLICATION

(11) **EP 3 144 393 A1**
(43) Date of publication of application: **22.03.2017**
(21) Application number: 15185823.0
(22) Date of filing: 18.09.2015
(51) Int. Cl.: C12P 17/10, C07C 315/00

(54) **A SYNTHETIC PATHWAY TOWARDS APREMILAST**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention relates to an asymmetric process for providing N-{2-[(1*S*)-1-(3-ethoxy-4-methoxyphenyl)-2-(methylsulfonyl)ethyl]-1,3-dioxo-2,3-dihydro-1*H*-isoindol-4-yl}acetamide (apremilast) or a pharmaceutically acceptable salt or solvate thereof.

## Description

### Field of the invention

The present invention relates to an enantioselective process for preparing apremilast or a pharmaceutically acceptable salt or solvate thereof through essentially enantiopure β-hydroxy halo or sulfone type intermediates.

### Background of the invention

Enantiomerically enriched β-hydroxy halo or sulfone type compounds and in particular an essentially enantiopure β-hydroxysulfone compound may provide useful intermediates for the synthesis of apremilast, an anti-inflammatory agent active for treatment of psoriatic arthritis. Apremilast, which is *N*-{2-[(1*S*)-1-(3-ethoxy-4-methoxyphenyl)-2-(methylsulfonyl)ethyl]-1,3-dioxo-2,3-dihydro-1*H*-isoindol-4-yl}acetamide, is an inhibitor of PDE4. Apremilast specifically inhibits PDE4 and spontaneous production of TNF-α from human rheumatoid synovial cells and has anti-inflammatory activity.

WO 2000/25777 A1 describes the synthesis of the racemate of apremilast and derivatives thereof. In WO 2010/030345 A2 a synthesis of apremilast is described which *inter alia* includes the formation of a benzonitrile derivative and furthermore a reduction step. These processes use reagents which are not satisfactory for industrial synthesis.

WO 2013/126360 A2 and WO 2013/126495 A2 describe processes to produce apremilast which include the preparation of enantiomerically pure chiral β-aminosulfone intermediates using expensive as well as toxic materials such as metal catalysts, ligands or chiral auxiliaries.

WO 2003/080049 A1 describes the optical resolution of a racemic β-aminosulfone intermediate using in particular *N*-acetyl-L-leucine to obtain apremilast.

The object of the present invention is to provide short, simple, cost-effective, environmentally friendly and industrially suitable processes for beneficially providing isoindoline-based compound apremilast or a pharmaceutically acceptable salt or solvate thereof.

### Summary of the invention

The object is solved by the methods according to claims 1, 2 and 14, the compounds according to claims 10, 11 and 12, and the use according to claim 13, while preferred embodiments are set forth in dependent claims and will be further described below.

Various aspects, advantageous features and preferred embodiments of the present invention as summarized in the following items, respectively alone or in combination, contribute to solving the object of the invention.
1. A process for asymmetrically synthesizing a compound of formula **6** or a pharmaceutically acceptable salt or solvate thereof 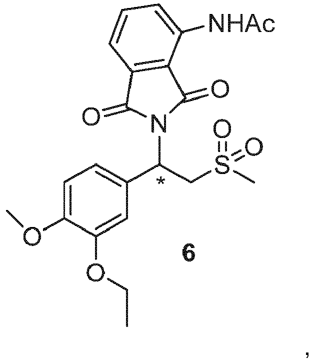 wherein * in the formulae denotes an asymmetric carbon atom in (*R*) or (*S*) configuration being enantiomerically enriched or essentially enantiopure, the process comprising the steps:
   (a) reducing a compound of formula **2** 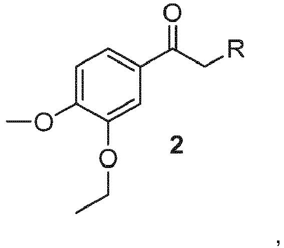 wherein R is methylsulfonyl -SO₂CH₃ or a group convertible to methylsulfonyl, by an asymmetric enzymatic reduction using baker's yeast containing fungi of the species *Saccharomyces cerevisiae* to give the compound of formula **3** 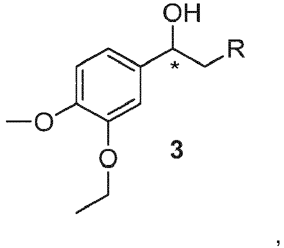 wherein the asymmetric enzymatic reduction leads to the (*R*) or the (*S*) configuration being enantiomerically enriched or essentially enantiopure,
   (b) if R is a group convertible to methylsulfonyl converting said group to methylsulfonyl -SO₂CH₃ group,
   (c) converting the compound of formula **3b** 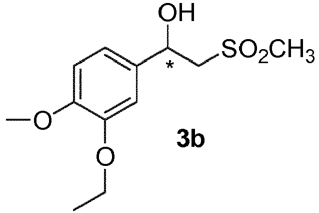 into the compound of formula **4** 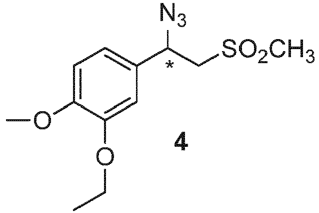
   (d) reducing the compound of formula 4 to obtain the compound of formula **5** 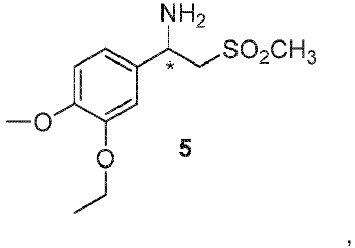 and
   (e) reacting the compound of formula 5 with 3-acetamidophthalic anhydride of formula 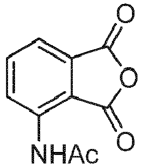 to obtain the compound of formula **6** or a pharmaceutically acceptable salt or solvate thereof.
   The term "enantiomerically enriched" as used herein means that one enantiomer predominates over the other expressing 10 to 70 % ee, preferably 30 to 70 % ee, more preferably 60 to 70 % ee.
   The term "essentially enantiopure" as used herein means an enantiomeric excess (ee) of 70 % ee or more, preferably 80 % ee or more, more preferably 90 % ee or more, most preferably 97 % ee or more.
   The term "a group convertible to methylsulfonyl" as used herein means any group, which can be transformed to methylsulfonyl by methods, known in the art, preferably in one chemical step. The group convertible to methylsulfonyl can be selected from the group consisting of methylsulfenyl, methylsulfinyl, -F, -Cl, -Br and -I. Preferably, the group convertible to methylsulfonyl is selected from the group consisting of -F, -Cl, -Br and -I. Most preferably the group convertible to methylsulfonyl is -Cl.
2. A process according to item 1, wherein the reduction of step (d) is carried out with triphenylphosphine in water.
3. A process for asymmetrically synthesizing a compound of formula **6** or a pharmaceutically acceptable salt or solvate thereof 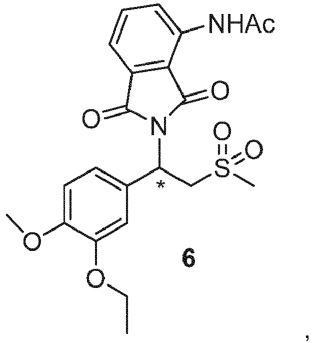 wherein * in the formulae denotes an asymmetric carbon atom in (*R*) or (*S*) configuration being enantiomerically enriched or essentially enantiopure, the process comprising the steps:
   (a) reducing a compound of formula **2** 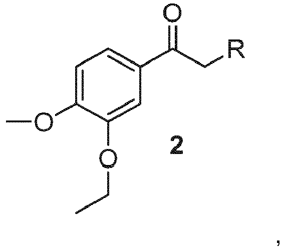 wherein R is methylsulfonyl -SO₂CH₃ or a group convertible to methylsulfonyl,
      by an asymmetric enzymatic reduction using baker's yeast containing fungi of the species *Saccharomyces cerevisiae* to give the compound of formula **3** 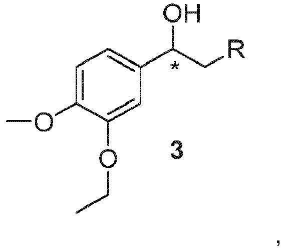 wherein the asymmetric enzymatic reduction leads to the (*R*) or the (*S*) configuration being enantiomerically enriched or essentially enantiopure,
   (b) if R is a group convertible to methylsulfonyl converting said group to methylsulfonyl -SO₂CH₃ group,
   (c) reacting the compound of formula **3b** 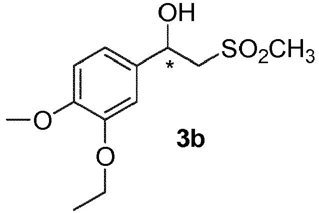 with 3-acetamidophthalimide of formula 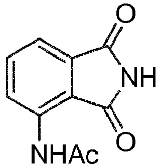 in the presence of a reagent selected from the group consisting of DEAD, DIAD and DCAD, in combination with Ph₃P and Bu₃P, to obtain the compound of formula **6** or a pharmaceutically acceptable salt or solvate thereof.
4. The process according to items 1 or 3, wherein the compound of formula **3** is produced with the asymmetric carbon atom indicated with * being enantiomerically enriched in the (*R*) configuration, preferably with an ee of at least 60 % ee.
5. The process according to item 4, wherein the compound of formula **3** with the asymmetric carbon atom indicated with * in the (*R*) configuration, is essentially enantiopure with an ee of at least 75 % ee.
6. The process according to item 5, wherein the compound of formula **3** with the asymmetric carbon atom indicated with * in the (*R*) configuration, is essentially enantiopure with an ee of at least 85 % ee.
7. The process according to item 1 or 3, wherein the baker's yeast containing fungi of the species *Saccharomyces cerevisiae* is in a water medium, optionally comprising glucose.
8. The process according to item 1 or 3, wherein the group convertible to methylsulfonyl is selected from the group consisting of methylsulfenyl, methylsulfinyl, -F, -Cl, -Br and -I.
9. The process according to item 8, wherein the group convertible to methylsulfonyl is selected from the group consisting of -F, -Cl, -Br and -I.
10. The process according to item 8 or 9, wherein the group convertible to methylsulfonyl is -Cl.
11. A compound of formula **2a** 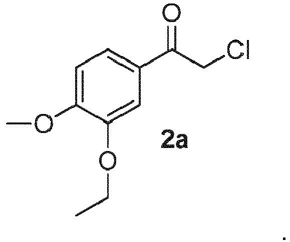
12. A compound of formula **3a** 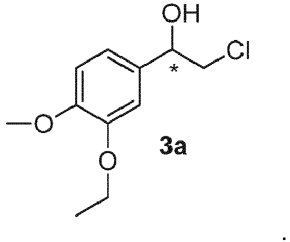 wherein * in the formulae denotes an asymmetric carbon atom in (*R*) or (*S*) configuration being enantiomerically enriched.
13. A compound of the formula **3a*R*** 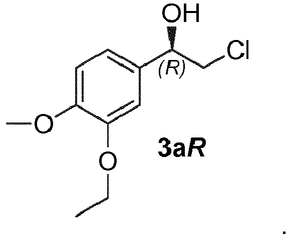
14. A compound of the formula **3a*S*** 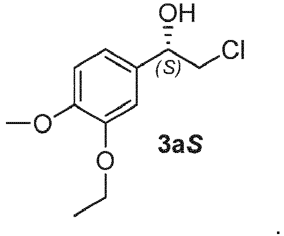
15. Use of the compound as defined in any of the items 11 to 13 in the preparation of *N*-{2-[(1*S*)-1-(3-ethoxy-4-methoxyphenyl)-2-(methylsulfonyl)ethyl]-1,3-dioxo-2,3-dihydro-1*H-*isoindol-4-yl}acetamide (apremilast) or a pharmaceutically acceptable salt or solvate thereof.
16. A process for the preparation of a pharmaceutical composition comprising *N*-{2-[(1*S*)-1-(3-ethoxy-4-methoxyphenyl)-2-(methylsulfonyl)ethyl]-1,3-dioxo-2,3-dihydro-1*H*-isoindol-4-yl}acetamide (apremilast) or a pharmaceutically acceptable salt or solvate thereof, comprising the steps of:
   x) preparing *N*-{2-[(1*S*)-1-(3-ethoxy-4-methoxyphenyl)-2-(methylsulfonyl)ethyl]-1,3-dioxo-2,3-dihydro-1*H*-isoindol-4-yl}acetamide (apremilast) or a pharmaceutically acceptable salt or solvate thereof by carrying out the process according to any of items 4 to 6, and
   y) mixing said obtained *N*-{2-[(1*S*)-1-(3-ethoxy-4-methoxyphenyl)-2-(methyl-sulfonyl)ethyl]-1,3-dioxo-2,3-dihydro-1*H*-isoindol-4-yl}acetamide (apremilast) or a pharmaceutically acceptable salt or solvate thereof with a pharmaceutically acceptable carrier and/or excipient.

### Detailed description

In the following, the present invention is described in more detail while referring to preferred embodiments and examples, which are presented however for illustrative purposes and shall not be construed to limit the invention in any way.

The present invention provides an industrially applicable, economical and simple enantioselective process for the preparation of apremilast or a pharmaceutically acceptable salt or solvate thereof. For apremilast, which is *N*-{2-[(1*S*)-1-(3-ethoxy-4-methoxyphenyl)-2-(methylsulfonyl)ethyl]-1,3-dioxo-2,3-dihydro-1*H*-isoindol-4-yl}acetamide, the synthetic route described herein benefits from simple reactions, mild reaction conditions and readily available and cheap chemicals. The starting compounds for the overall synthesis are readily available by simple processes known to a skilled person. The chiral reduction of carbonyl-containing intermediate according to the invention applies enzymatic approach with easy available and cheap enzymes leading to corresponding chiral β-hydroxy intermediates with high ee. Such intermediates are easily converted to chiral *N*-{2-[1-(3-ethoxy-4-methoxyphenyl)-2-(methylsulfonyl)ethyl]-1,3-dioxo-2,3-dihydro-1*H*-isoindol-4-yl}acetamide with a desired chirality.

A first aspect of the invention is a process for providing a compound of formula **6**

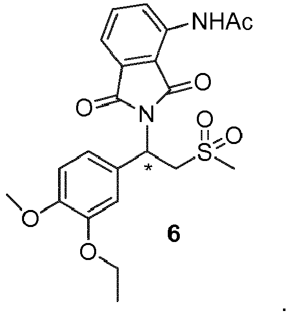

Said process presented in Scheme 1 starts from a commercially available compound 1-(3-ethoxy-4-methoxyphenyl)ethanone (1), which can be converted to the compound of formula **2',** in which X represents a halogen, by implementing a suitable halogenating agent. Halogen can be introduced via electrophilic halogenation, for example, with respective N-halo succinimide such as N-chlorosuccinimide (NCS), N-bromosuccinimide (NBS), N-iodosuccinimide (NIS), or with reagents such as 1,3-dibromo-5,5-dimethylhydantoin and the like. Alternatively, the halogen can be introduced with oxidative halogenation, for example, with HBr/H₂O₂, KCl/acid/H₂O₂, Kl/acid/H₂O₂, etc. Next, the compound of formula **2'** is asymmetrically reduced to the compound of formula **3'** in the presence of the baker's yeast containing fungi of the species *Saccharomyces cerevisiae* in a suitable solvent, for example, in water. The compound of formula **3'** is subsequently converted to the compound of formula **3b** by implementing a suitable sulfinating agent, for example, methanesulfonyl chloride in dichloromethane. Alternatively the compound of formula **2'** can be first converted to the compound of formula **2b** by implementing a suitable sulfinating agent, for example, MeSO₂Na in ethanol, and **2b** is subsequently asymmetrically reduced to the compound of formula **3b** in the presence of the baker's yeast containing fungi of the species *Saccharomyces cerevisiae* in a suitable solvent, for example, in water.

The compound of formula **3b** is converted to the compound of formula 6 in several steps. First, **3b** is contacted with a suitable solvent, for example, toluene and THF and then the mixture is flushed with N₂. This is followed by adding a solution of HN₃ in toluene and cooling the so obtained mixture. Once a temperature below -70°C is reached, tributylphosphine (Bu₃P) and diethylazodicarboxylate (DEAD) are added and the mixture is stirred at a temperature below - 70°C for four hours. The mixture is then let to reach the room temperature, the solvents are evaporated and the obtained residue is dissolved in ethyl acetate and washed with brine and heptane to yield the compound of formula **4.** Next, the obtained compound of formula **4** is dissolved in THF and contacted with triphenylphosphine (Ph₃P) in water and stirred under N₂ atmosphere overnight. After solvent evaporation, ethyl acetate is added to a two-phase mixture of water and yellow oil and the mixture is washed with brine. Na₂SO₄ is used to dry the organic phase to yield the compound of formula **5**. In the last step the obtained compound of formula **5** is converted to the compound of formula **6** by following processes known in the art, for example, referring to WO 00/25777 A1 and WO 03/080049 A1, **6** can be obtained in a synthesis using 3-acetamidophthalimide, e.g. carried out in acetic acid.

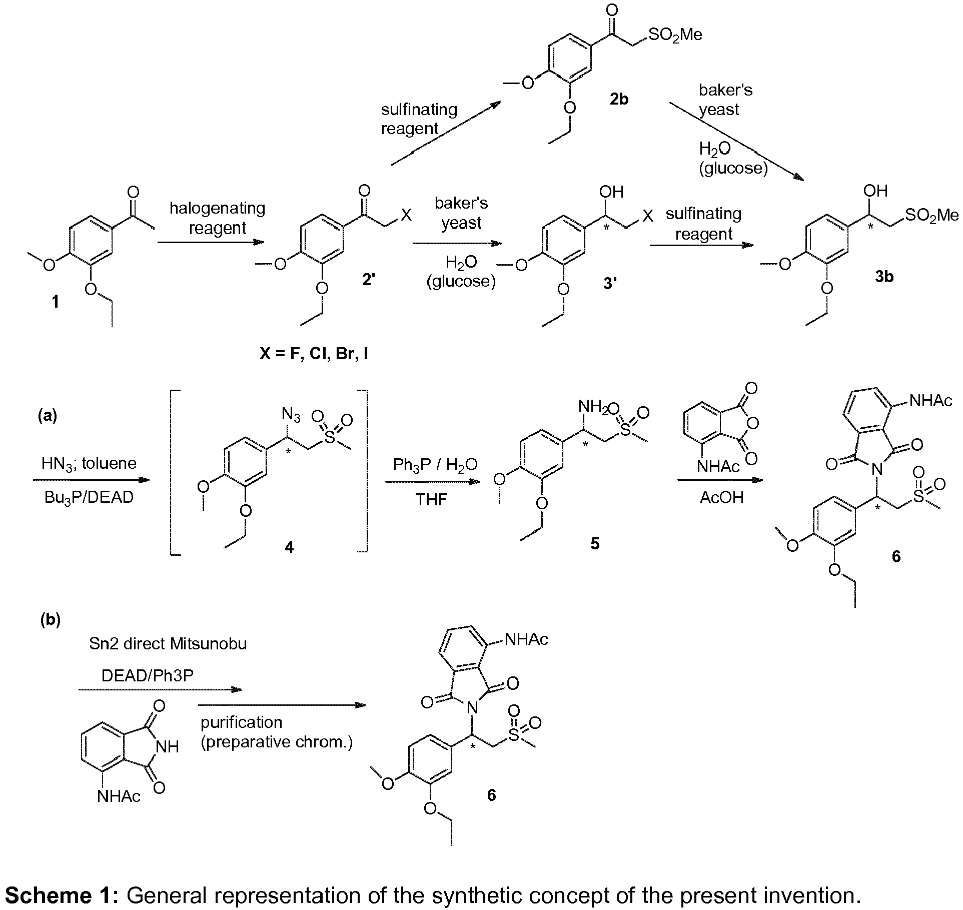

Alternatively (as presented in Scheme 1 - (b)), the compound of formula **6** can be obtained by a direct coupling of the compound of formula **3b** with 3-acetamidophthalimide in the presence of an azodicarboxylate type reagent selected from the group consisting of DEAD (diethyl azodicarboxylate), DIAD (diisopropyl azodicarboxylate), and DCAD (di(4-chlorobenzyl) azodicarboxylate), in combination with Ph₃P and Bu₃P.

Due to the high efficiency of the asymmetric reduction, the preferred type of baker's yeast is the one containing fungi of the species *Saccharomyces cerevisiae.* The yeast is prepared in a water medium. The mixture is optionally pre-prepared by adding a feed, preferably selected from glucose tempering it at the temperature from 15 to 40 °C, preferably at 30 °C. A substrate selected from a compound according to the formula **2,** i.e. **2'** or **2b,** is added undissolved or dissolved in a microorganism friendly water miscible solvent, preferably selected from alcohols or acetone, most preferably ethanol. The bioreaction mixture is usually stirred for 3 hours to 5 days, preferably 2 days, at the temperature from 15 to 40 °C, preferably at 30 °C. The product can be isolated by removal of biomaterial followed by extraction and can be purified by the methods of state of the art.

In a specific embodiment presented in Scheme 2, the compound 1-(3-ethoxy-4-methoxyphenyl)-ethanone (1) is converted to the compound of formula **2a** using N-chlorosuccinimide (NCS) and p-toluenesulfonic acid (p-TsOH) in acetonitrile (MeCN). The compound of formula **2a** is asymmetrically reduced to the compound of formula **3a** in the presence of the baker's yeast containing fungi of the species *Saccharomyces cerevisiae* in water or in solution of water and glucose. The obtained essentially enantiopure intermediate **3a** is further converted to **3b** by reacting **3a** with methanesulfonyl chloride in dichloromethane.

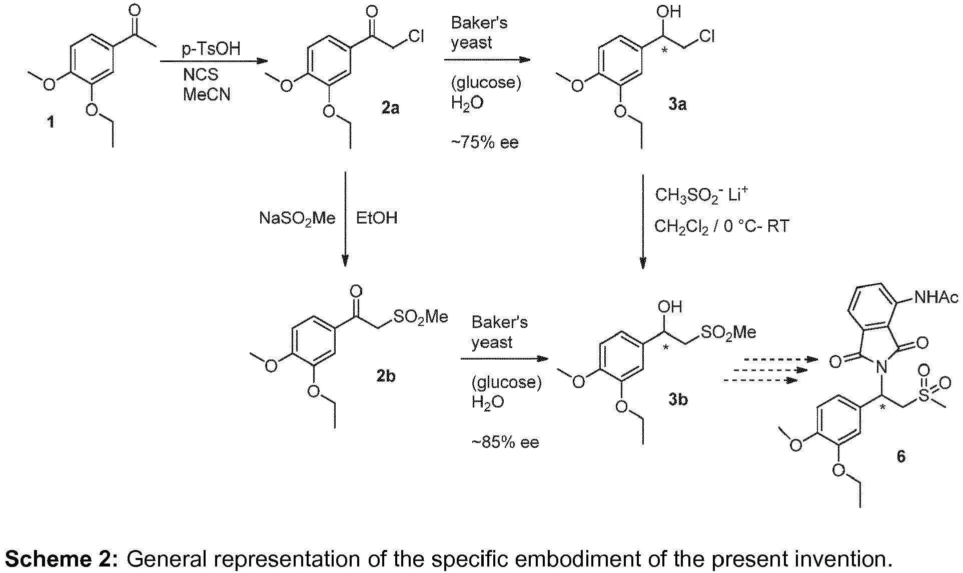

Alternatively, the compound of formula **2a** can be first converted to the compound of formula **2b** with MeSO₂Na in ethanol. The essentially enantiopure intermediate **3b** is obtained by asymmetric reduction of **2b** in the presence of the baker's yeast containing fungi of the species *Saccharomyces cerevisiae* in water or in solution of water and glucose.
In the final steps, the compound of formula **6** is produced according to the procedure(s) explained in Scheme 1 above.

The purity of a chiral compound is measured by enantiomeric excess (ee), which is a degree to which one enantiomer is contained in a greater amount in a sample than the other enantiomer. Specifically, the enantiomeric excess is defined as the difference between the mole fractions of each enantiomer. Preferably, the one enantiomer provided by the process is obtained in an enantiomeric excess of at least 20%, more preferably at least 40%, even more preferably at least 60%, still more preferably at least 80%, yet more preferably at least 90%. Still more preferably the enantiomer obtained by the process is purified to at least 95% ee. It is particularly preferred that the enantiomer obtained by the process is pure. Pure enantiomer means that the one optical isomer of a compound is substantially free, preferably free, of the other enantiomer of the compound. Typically a pure enantiomer comprises greater than 98 wt.% of the one enantiomer and less than 2 wt.% of the other enantiomer, preferably greater than 99 wt.% of the one enantiomer and less than 1 wt.% of the other enantiomer.

In another aspect of the present invention a process is provided for preparing apremilast, i.e. *N-*{2-[(1*S*)-1-(3-ethoxy-4-methoxyphenyl)-2-(methylsulfonyl)ethyl]-1,3-dioxo-2,3-dihydro-1*H-*isoindol-4-yl}acetamide or a pharmaceutically acceptable salt or solvate thereof.

More specifically, apremilast can be prepared from the compound of formula **3b*R*** by following a process presented in Scheme 3.

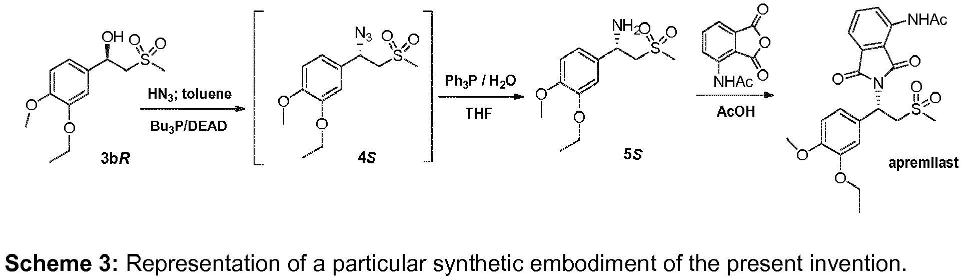

In the first step, the compound of formula **3b*R*** is contacted with a suitable solvent, for example, toluene and THF and then the mixture is flushed with N₂. This is followed by adding a solution of HN₃ in toluene and cooling the so obtained mixture. Once a temperature below -70°C is reached, tributylphosphine (Bu₃P) and diethylazodicarboxylate (DEAD) are added and the mixture is stirred at a temperature below -70°C for four hours. The mixture is then let to reach the room temperature, the solvents are evaporated and the obtained residue is dissolved in ethyl acetate and washed with brine and heptane to yield the compound of formula **4*S*.** Next, the obtained compound of formula **4*S*** is dissolved in THF and contacted with triphenylphosphine (Ph₃P) in water and stirred under N₂ atmosphere overnight. After solvent evaporation, ethyl acetate is added to a two-phase mixture of water and yellow oil and the mixture is washed with brine. Na₂SO₄ is used to dry the organic phase to yield a yellow solid residue. After another round of purification, finally white crystals of the compound of formula **5*S*** are obtained.
By following said process, the compound of formula **5*S*** with ee of around 80% can be obtained. To further improve the optical purity, *N*-acetyl-L-leucine may be utilized to give a stereo-chemically pure compound of formula **5*S*** with an ee of around 99%.
In the last step the obtained compound of formula **5*S*** is converted to apremilast by following processes known in the art, for example, referring to WO 00/25777 A1 and WO 03/080049 A1, apremilast can be obtained in a synthesis using 3-acetamidophthalimide and the chiral amino acid salt of the (*S*) enantiomer of the β-aminosulfone compound of formula **5*S*,** e.g. carried out in acetic acid.

Alternatively, apremilast can be prepared directly from the compound of formula **3b*R*** by following the Mitsunobu reaction as presented in Scheme 4. The compound of formula **3b*R*** is coupled with 3-acetamidophthalimide in the presence of an azodicarboxylate type reagent selected from the group consisting of DEAD (diethyl azodicarboxylate), DIAD (diisopropyl azodicarboxylate), and DCAD (di(4-chlorobenzyl) azodicarboxylate), in combination with Ph₃P and Bu₃P to yield crude apremilast. Using preparative chromatography as a purification method, pure apremilast can be obtained.

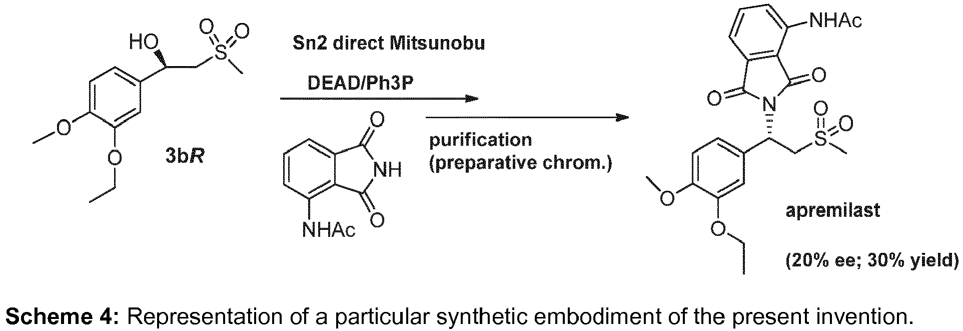

Further embodiment of the present invention resides in the provision of a valuable intermediate compounds **2a, 3a** and **3a*R*** useful in the synthesis of apremilast

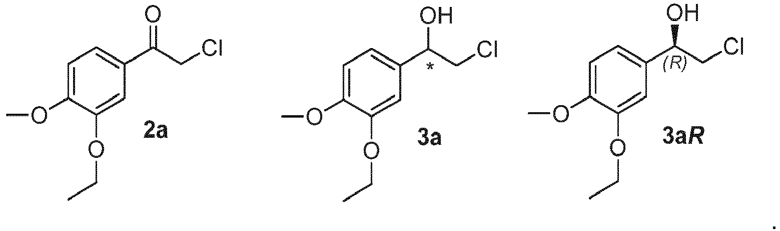

Another aspect of the invention is a process for preparing a pharmaceutical composition comprising apremilast or a pharmaceutically acceptable salt or solvate thereof, the process comprising the steps of carrying out the process of the invention to obtain apremilast or a pharmaceutically acceptable salt or solvate thereof, and mixing apremilast or a pharmaceutically acceptable salt or solvate thereof obtained according to the invention, optionally with another active pharmaceutical ingredient, with a pharmaceutically acceptable excipient, carrier and/or diluent.

In particular, apremilast or a pharmaceutically acceptable salt or solvate thereof is admixed with at least one suitable pharmaceutically acceptable excipient. Pharmaceutically acceptable excipients may be selected from the group consisting of diluents, carriers, binders, disintegrating agents, stabilizing agents, preservatives, lubricants, surfactants, fragrances, flavouring agents, sweeteners and other excipients known in the field of the pharmaceutical technology. For example, suitable excipients may be selected from the group consisting of lactose, microcrystalline cellulose, cellulose derivatives, such as hydroxypropylcellulose, polyacrylates, calcium carbonate, starch, colloidal silicone dioxide, sodium starch glycolate, croscarmellose sodium, talc, magnesium stearate, polyvinylpyrrolidone, polyethylene glycol and other excipients known in the field of the pharmaceutical technology.

Overall, in the present invention a simple and robust enantioselective synthesis of *N*-{2-[1-(3-ethoxy-4-methoxyphenyl)-2-(methylsulfonyl)ethyl]-1,3-dioxo-2,3-dihydro-1*H*-isoindol-4-yl}acetamide **(6)** is provided. The essentially enantiopure β-hydroxy sulfone intermediate **(3b)** is used in a process to obtain **6** using simple and robust chemical transformations. Simple and commercially available reagents and catalysts are used, and the intermediates may be formed directly without the need for additional protection/deprotection steps. Hazardous, toxic and expensive chemicals can favourably be avoided. In addition, there is no need for cumbersome preparatory equipment or techniques. Overall, the present invention thus provides cost-effective, environmentally friendly and industrially suitable synthetic processes.

### Examples

The following examples are merely illustrative of the present invention and they should not be considered as limiting the scope of the invention in any way. The examples and modifications or other equivalents thereof will become apparent to those versed in the art in the light of the present entire disclosure.

### Example 1: Synthesis of 2-chloro-1-(3-ethoxy-4-methoxyphenyl)ethanone (2a)

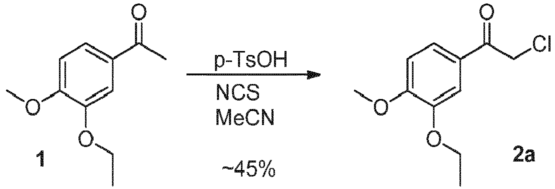

N-Chlorosuccinimide (15.45 mmol, 2.1 g) was added to a solution of 1-(3-ethoxy-4-methoxyphenyl)ethanone (10.3 mmol, 2.0 g) and p-TsOH x H₂O (10.3 mmol, 2.0 g) in MeCN (40 mL). The reaction mixture was stirred overnight at 40 °C. After removal of solvent, the obtained crude product was purified by flash chromatography (silica gel column, mobile phase: n-heptane/EtOAc, EtOAc gradient 5%-40%) to give **2a** in 45% yield.
¹H NMR (500 MHz, CDCl₃, ppm) δ 7.57-7.53 (m, 2H), 6.92 (d, *J* = 8.4 Hz, 1H), 4.67 (s, 2H), 4.17 (q, *J* = 7.5 Hz, 2H), 3.97 (s, 3H), 1.50 (t, *J* = 7.1 Hz, 3H).

### Example 2: Synthesis of 1-(3-ethoxy-4-methoxyphenyl)-2-(methylsulfonyl)ethanone (2b)

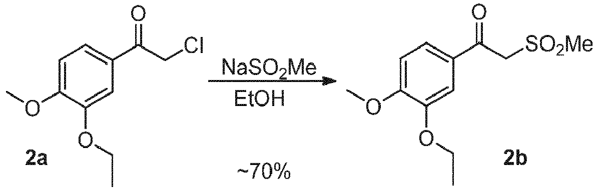

2-Chloro-1-(3-ethoxy-4-methoxyphenyl)ethanone (**2a**, 2.19 mmol, 0.5 g) was added to a suspension of MeSO₂Na (2.41 mmol, 0.26 g) in EtOH (5 mL). The reaction mixture was stirred under reflux for 3 hours. After cooling to room temperature, the reaction mixture was poured into a beaker with ice-water. The crude product was isolated from reaction mixture by extraction with CH₂Cl₂ followed. After drying over MgSO₄ and solvent removal, the pure **2b** was obtained by flash chromatography (silica gel column, mobile phase: n-heptane/EtOAc, EtOAc gradient 7%-60%) in 70% yield.
¹H NMR (500 MHz, CDCl₃, ppm) δ 7.63 (dd, *J* = 8.6, *J* = 2.1 Hz, 1 H), 7.55 (ds, *J* = 2.0 Hz, 1 H), 6.95 (d, *J* = 8.5 Hz, 1 H), 4.57 (ds, *J* = 0.6 Hz, 2H), 4.18 (q, *J* = 7.1 Hz, 2H), 3.98 (s, 3H), 3.61 (s, 3H), 1.51 (t, *J* = 6.9 Hz, 3H);

### Example 3: Synthesis of 2-chloro-1-(3-ethoxy-4-methoxyphenyl)ethanol (3a) by baker's yeast mediated asymmetric reduction (method A)

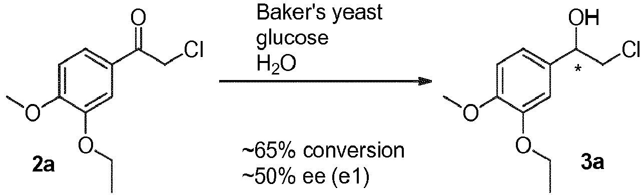

Into a beaker were placed 2-chloro-1-(3-ethoxy-4-methoxyphenyl)ethanone (**2a**, 0.66 mmol, 150 mg), baker's yeast containing fungi of the species *Saccharomyces cerevisiae* (6 g), glucose (6 g) and water (120 mL). The mixture was stirred at room temperature for 48 hours. Celite® was added and the mixture was stirred for 15 min. After filtration, the cake was washed several times with EtOAc. Obtained filtrate phases were separated and water phase was washed with EtOAc. Combined organic phases were dried over MgSO₄, filtered and solvent was removed by evaporation. The crude product (65 % conversion was determined by ¹H NMR) was purified by flash chromatography (silica gel column, mobile phase: n-heptane/EtOAc, EtOAc gradient 7%-60%) to give **3a** (50.61% e.e. (e1) by chiral HPLC).
¹H NMR (500 MHz, CDCl₃, ppm) δ 6.94-6.85 (m, 3H), 4.85-4.82 (m, 1H), 4.14-4.09 (m, 2H), 3.87 (s, 3H), 3.72-3.61 (m, 2H), 1.47 (t, *J* = 7.2, 3H).

### Example 4: Synthesis of 2-chloro-1-(3-ethoxy-4-methoxyphenyl)ethanol (3a) by baker's yeast mediated asymmetric reduction (method B)

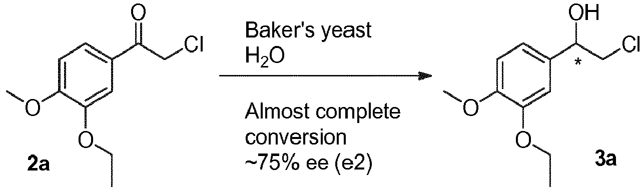

Into a beaker were placed 2-chloro-1-(3-ethoxy-4-methoxyphenyl)ethanone (**2a**, 0.22 mmol, 50 mg), baker's yeast containing fungi of the species *Saccharomyces cerevisiae* (8 g) and water (20 mL). The mixture was stirred at 30 °C for 48 hours. Celite® was added and the mixture was stirred for 15 min. After filtration, the cake was washed several times with EtOAc. Obtained filtrate phases were separated and water phase was washed with EtOAc. Combined organic phases were dried over MgSO₄, filtered and solvent was removed by evaporation. The crude product (almost complete conversion was determined by ¹H NMR, 75.72% e.e. (e2) by chiral HPLC) can be purified by flash chromatography (silica gel column, mobile phase: n-heptane/EtOAc, EtOAc gradient 7%-60%) to give **3a.**
¹H NMR (500 MHz, CDCl₃, ppm) δ 6.94-6.85 (m, 3H), 4.85-4.82 (m, 1H), 4.14-4.09 (m, 2H), 3.87 (s, 3H), 3.72-3.61 (m, 2H), 1.47 (t, *J* = 7.2, 3H).

### Example 5: Synthesis of 1-(3-ethoxy-4-methoxyphenyl)-2-(methylsulfonyl)ethanol (3b) by baker's yeast mediated asymmetric reduction (method A)

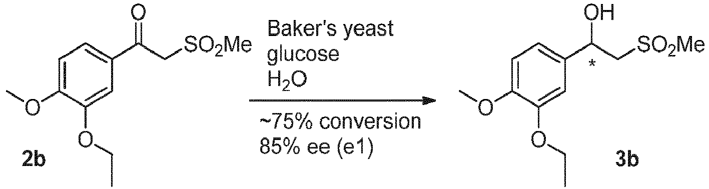

Into a beaker were placed 1-(3-ethoxy-4-methoxyphenyl)-2-(methylsulfonyl)ethanone (**2b**, 0.18 mmol, 50 mg), baker's yeast containing fungi of the species *Saccharomyces cerevisiae* (6 g), glucose (2 g) and water (20 mL). The mixture was stirred at 30 °C for 48 hours. Celite® was added and the mixture was stirred for 15 min. After filtration, the cake was washed several times with EtOAc. Obtained filtrate phases were separated and water phase was washed with EtOAc. Combined organic phases were dried over MgSO₄, filtered and solvent was removed by evaporation. The crude product (75 % conversion was determined by ¹H NMR, 85.56% e.e. (e1) by chiral HPLC) can be purified by flash chromatography (silica gel column, mobile phase: n-heptane/EtOAc, EtOAc gradient 12%-100%) to give **3b.**
¹H NMR (500 MHz, CDCl₃, ppm) δ 6.92-6.86 (m, 3H), 5.29 (td, *J* = 10.4 Hz, *J* = 2.1 Hz, 1H), 4.12 (q, *J* = 6.7, 2H), 3.88 (s, 3H), 3.49-3.44 (m, 1 H), 3.19-3.16 (m, 1 H), 3.06 (s, 3H), 1.49 (t, *J =* 7.1, 3H).

### Example 6: Synthesis of 1-(3-ethoxy-4-methoxyphenyl)-2-(methylsulfonyl)ethanol (3b) by baker's yeast mediated asymmetric reduction (method B)

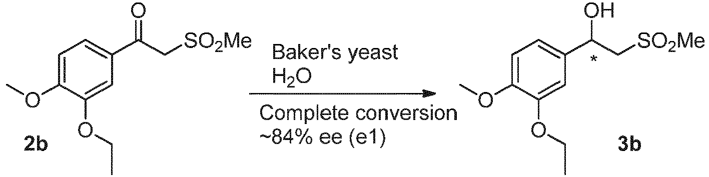

Into a beaker were placed 1-(3-ethoxy-4-methoxyphenyl)-2-(methylsulfonyl)ethanone (**2b**, 0.18 mmol, 50 mg), baker's yeast containing fungi of the species *Saccharomyces cerevisiae* (6 g), glucose (2 g) and water (20 mL). The mixture was stirred at 30 °C for 48 hours. Celite® was added and the mixture was stirred for 15 min. After filtration, the cake was washed several times with EtOAc. Obtained filtrate phases were separated and water phase was washed with EtOAc. Combined organic phases were dried over MgSO₄, filtered and solvent was removed by evaporation. The crude product (complete conversion was determined by ¹H NMR, 85.2% e.e. (e1) by chiral HPLC) was purified by flash chromatography (silica gel column, mobile phase: n-heptane/EtOAc, EtOAc gradient 12%-100%) to give **3b.**
¹H NMR (500 MHz, CDCl₃, ppm) δ 6.92-6.86 (m, 3H), 5.29 (td, *J* = 10.4 Hz, *J* = 2.1 Hz, 1H), 4.12 (q, *J =* 6.7, 2H), 3.88 (s, 3H), 3.49-3.44 (m, 1 H), 3.19-3.16 (m, 1 H), 3.06 (s, 3H), 1.49 (t, *J =* 7.1, 3H).

### Example 7: Synthesis of 1-(3-ethoxy-4-methoxyphenyl)-2-(methylsulfonyl)ethanol (3b) from 2-chloro-1-(3-ethoxy-4-methoxyphenyl)ethanol (3a)

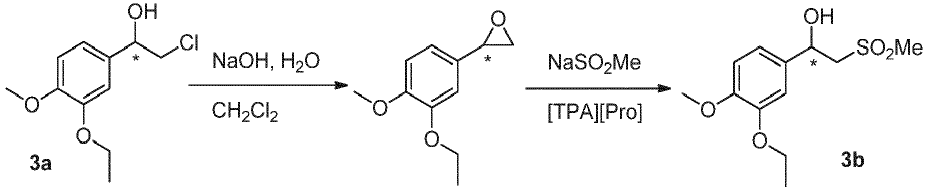

A solution of 2-chloro-1-(3-ethoxy-4-methoxyphenyl)ethanol **(3a)** in CH₂Cl₂ and a 2M aqueous solution of NaOH is stirred at room temperature for several hours. To the solution is added additional amount of CH₂Cl₂ and the layers are separated. The organic phase is washed with a saturated solution of NaCl and dried over MgSO₄. The solvent is removed by evaporation and the residue is purified by chromatography to give 2-(3-ethoxy-4-methoxyphenyl)oxirane. The latter is mixed with MeSO₂Na in ionic liquid [TPA][Pro] and stirred first at room temperature for several hours and then at 80 °C for several hours. After cooling down to room temperature, water is added and the product is extracted into EtOAc. Organic phase is washed with saturated solution of NaCl, dried over MgSO₄ and the solvent is removed by evaporation. The residue is purified by chromatography to give **3b.**

### Example 8: Synthesis of 1-(3-ethoxy-4-methoxyphenyl)-2-(methylsulfonyl)ethanol (3b) from 2-chloro-1-(3-ethoxy-4-methoxyphenyl)ethanol (3a)

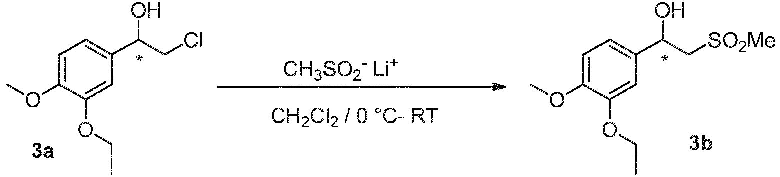

Into a test tube equipped with magnetic stirrer and septum was placed starting material **(3a)** (1 mmol) which was dissolved in dry CH₂Cl₂ (3.5 mL) and solution was cooled down to 0 °C. Afterwards alkali salt of methanesulfonyl chloride (1.5 mmol) was slowly added in portions and reaction mixture was stirred for two hours at 0 °C and afterwards intensively stirred at room temperature overnight. To this solution water was added to quench the reaction and then extracted with EtOAc (2 x 30 mL). The combined organic phases were washed with brine and dried over anhydrous Na₂SO₄. The solvent was evaporated under reduced pressure and obtained crude product was finally purified with flash chromatography (SiO₂; EtOAc/ n-hexane 1 : 10) to give **3b** which was confirmed with 1H NMR analysis.

### Example 9: Preparation of compound 4S

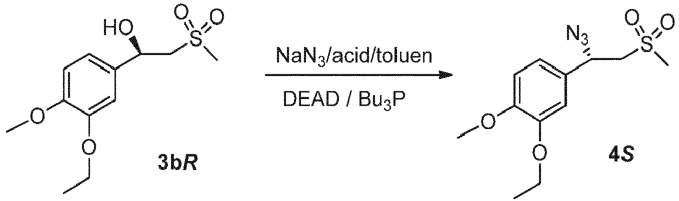

Optically active 1-(3-ethoxy-4-methoxyphenyl)-2-(methylsulfonyl)ethanol **(3b*R*)** (0.20 mg; 0.73 mmol) was weighed into a three-necked round-bottom flask, equipped with a thermometer. Toluene (56 mL) and fresh THF (4 mL) were added and the mixture was flushed with N₂ for 10 minutes. Freshly prepared solution of HN₃ in toluene (4.4 mL; 4.38 mmol) was added and the mixture was cooled in ethyl acetate - liquid N₂ bath. When the internal temperature reached less than -70° C, tributylphosphine (0.36 mL; 1.46 mmol) and then a solution of diethyl azodicarboxylate in toluene (0.77 mL; 1.68 mmol) were added and the mixture was stirred for additional 4 hours below -70 °C. After additional 18 hours the temperature reached room temperature. The solvents were evaporated under reduced pressure and the yellow oily residue was dissolved in 25 mL of ethyl acetate and washed three times with 25 mL of brine. The organic layer was dried over Na₂SO₄, filtered and the solvent was evaporated under reduced pressure to obtain yellow oil, which slowly solidified at room temperature. The solid residue was then triturated with 5 mL of heptane to obtain white crystals. The product **4*S*** (76% of isolated yield) was confirmed with ¹H NMR analysis and was used without additional purification in the next step using telescoping approach.
¹H NMR (500 MHz, CDCl₃) δ = 1.51 (t; *J* = 7.0 Hz; 3H; CH₂C*H*₃), 2,99 (s; 3H; SO₂CH₃), 3.17 (dd; *J* = 14.9 Hz, 3.7 Hz; 1 H; CHₐ), 3,45 (dd; *J =* 14.9 Hz, 9.9 Hz; 1 H; CH_{b}), 3.91 (s; 3H; OCH₃), 4.14 (q; *J* = 7.0 Hz; 2H; C*H*₂CH₃), 5.08 (dd; *J* = 9.9 Hz, 3.7 Hz; 1 H; CHN₃), 6.84 (s; 1 H; C₍₅₎H), 6.92 (s; 2H; C₍₂₎H, C₍₆₎H).

### Example 10: Synthesis of optically active 1-(3-ethoxy-4-methylphenyl)-2-(methylsulfonyl)-ethanamine (5S) using triphenylphoshine in water

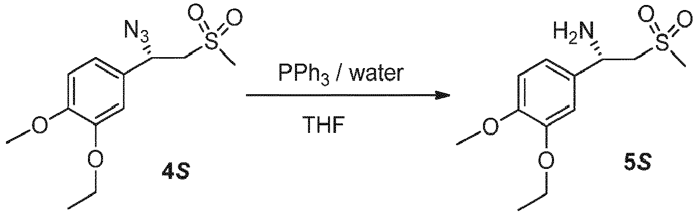

Triphenylphosphine (0.29 mg; 1.10 mmol) was weighed into a 50-mL round-bottom flask. Crude optical active compound **4*S*** (0.39 mg; 0.73 mmol) was dissolved in 10 mL of THF and added to the flask. Afterwards water (1 mL) was added and then the flask was sealed with a rubber septum and reaction mixture was stirred under N₂ atmosphere overnight. Afterwards solvent was evaporated under reduced pressure to obtain a two phase mixture of water and yellow oil. Ethyl acetate (20 mL) was added and washed twice with 20 mL of brine. The organic layer was dried over Na₂SO₄, filtered and evaporated under reduced pressure to obtain yellow solid residue. NMR analysis indicated the presence of iminophosphorane intermediate. Therefore the residue was dissolved in 10 mL of THF and 1 mL of water, transferred the mixture to a 50-mL round bottom flask equipped with a reflux condenser and stirred under reflux overnight. The solvent was evaporated under reduced pressure, 20 mL of ethyl acetate was added to the residue and washed three times with 10 mL of 1 M HCl. Combined aqueous layers were neutralized with K₃PO₄ until the pH was approximately 9 and then washed three times with 15 mL of ethyl acetate. The last three organic layers were combined, dried over Na₂SO₄, filtered off and evaporated under reduced pressure to obtain white crystals with 55% of isolated yield. Material was than dissolved in 1 mL of methanol and was heated to 80 °C over a period of 30 min. When the temperature reached between 35 and 40 °C, *N*-acetyl-L-leucine (0.6 equiv.) was added and the vial was sealed with a metal cap with septum. The mixture was stirred at 80 °C for 1 h and then cooled down to 20 °C in 2-3 hours. The white suspension was filtered through a Hirsch funnel (pore size No. 4) to obtain white solid material which was neutralized using NaOH in MTBE. Chiral HPLC analysis confirmed (*S*)-1-(3-ethoxy-4-methylphenyl)-2-(methylsulfonyl)ethanamine (**5*S***) with 99% optical purity. Compound **5*S*** was confirmed also with ¹H NMR spectroscopy.
¹H NMR (500 MHz, CDCl₃) δ = 1.48 (t; *J* = 7.0 Hz; 3H; CH₂C*H*₃), 2.92 (s; 3H; SO₂CH₃), 3.23 (dd; *J =* 14.1 Hz, 3.3 Hz; 1 H; CHₐ), 3.34 (dd; *J =* 14.1 Hz, 9.5 Hz; 1 H; CH_{b}), 3.87 (s; 3H; OCH₃), 4.11 (q; *J* = 7.0 Hz; 2H; C*H*₂CH₃), 4.61 (dd; *J* = 9.5 Hz, 3.3 Hz; 1 H; C*H*NH₂), 6.85 (d; *J* = 8.2 Hz; 1 H; C₍₅₎H), 6.91 (dd; *J* = 8.2 Hz, 2.0 Hz; 1 H; C₍₆₎H), 6.93 (d; *J* = 2.0 Hz; 1 H; C₍₂₎H)

### Example 11: Preparation of (S)-1-(3-ethoxy-4-methoxyphenyl)-2-methylsulfonylethyl)-4-amino-isoindoline-1,3-dione (apremilast) from the compound of formula 5S

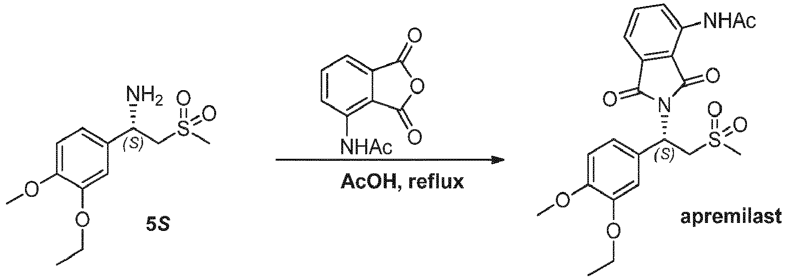

Into a reaction vessel equipped with magnetic stirrer and condenser was placed (S)-1-(3-ethoxy-4-methoxyphenyl)-2-(methylsulfonyl)ethanamine (2.24 mmol, 1 g) and was dissolved in 10 mL of glacial acetic acid. Afterwards 3-acetamidophthalic anhydride was added (2.35 mmol, 0.48 g) and reaction mixture was vigorously stirred at reflux overnight. Reaction system was cooled down to room temperature and acetic acid was evaporated under reduced pressure. The organic residue was extracted with dichloromethane, organic phases were washed with water, dried over Na₂SO₄ and solvent was evaporated. The residue was recrystallized from acetone/ethanol mixture, solid material was filtered off and dried in *vacuo* at 60 °C affording 0.66 g (64% Yield) of final product with > 99% ee. ¹H NMR analysis was in agreement with known data.

### Example 12: Direct transformation of optically active 1-(3-ethoxy-4-methoxyphenyl)-2-(methylsulfonyl)ethanol (3bR) to N-{2-[(1S)-1-(3-ethoxy-4-methoxyphenyl)-2-(methylsulfonyl)ethyl]-1,3-dioxo-2,3-dihydro-1H-isoindol-4-yl}acetamide (apremilast)

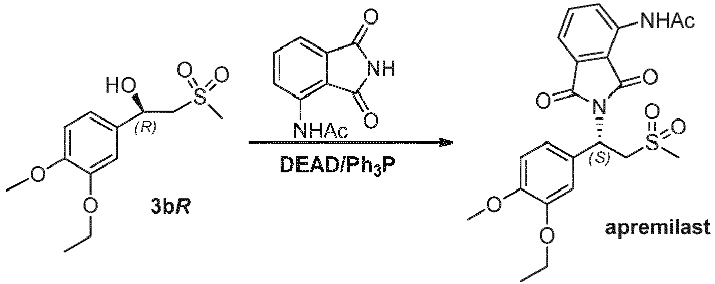

A 50 mL round bottom flask was charged with triphenylphosphine (1.75 mmol, 1.75 eq) and dichloromethane (3.4 mL). Afterwards 3-acetamidophthalimide (1 mmol, 1 eq) was added and the mixture was mixed at room temperature (rt). After 15 min optical active starting material 1-(3-ethoxy-4-methoxyphenyl)-2-(methylsulfonyl)ethanol (1 mmol, 1 eq; 98% optical purity) was added and the reaction mixture was cooled on ice. Later DEAD (40 % solution in toluene; 1.75 mmol, 1.75 eq) was added slowly and the mixture was mixed overnight at rt.
The following day a small amount of dichloromethane was added to the mixture, washed with brine and organic phase was dried over Na₂SO₄. The organic phase was evaporated in vacuo and 1.4 g of oily crude material was obtained. Crude material was diluted in ethylacetate and purified by column chromatography (SiO₂,EtOAc:n-heptane gradient elution) to afford yellowish solid apremilast (*N*-{2-[(1*S*)-1-(3-ethoxy-4-methoxyphenyl)-2-(methylsulfonyl)ethyl]-1,3-dioxo-2,3-dihydro-1*H*-isoindol-4-yl}acetamide) (22% yield) with 20% ee measured by chiral HPLC. The compound *N*-{2-[(1*S*)-1-(3-ethoxy-4-methoxyphenyl)-2-(methylsulfonyl)ethyl]-1,3-dioxo-2,3-dihydro-1*H*-isoindol-4-yl}acetamide was confirmed also with ¹H NMR spectroscopy.

## Claims

1. A process for asymmetrically synthesizing a compound of formula **6** or a pharmaceutically acceptable salt or solvate thereof 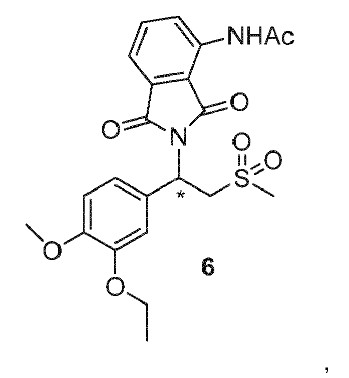 wherein * in the formulae denotes an asymmetric carbon atom in (*R*) or (*S*) configuration being enantiomerically enriched or essentially enantiopure, the process comprising the steps:
(a) reducing a compound of formula **2** 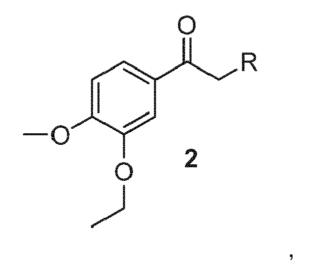 wherein R is methylsulfonyl -SO₂CH₃ or a group convertible to methylsulfonyl,
by an asymmetric enzymatic reduction using baker's yeast containing fungi of the species *Saccharomyces cerevisiae* to give the compound of formula **3** 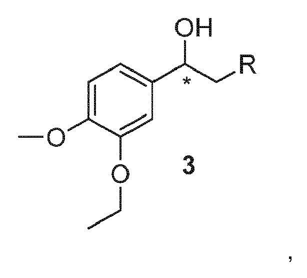 wherein the asymmetric enzymatic reduction leads to the (*R*) or the (*S*) configuration being enantiomerically enriched or essentially enantiopure,
(b) if R is a group convertible to methylsulfonyl converting said group to methylsulfonyl -SO₂CH₃ group,
(c) converting the compound of formula **3b** 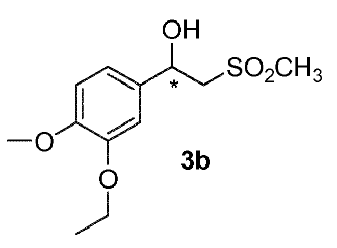 into the compound of formula **4** 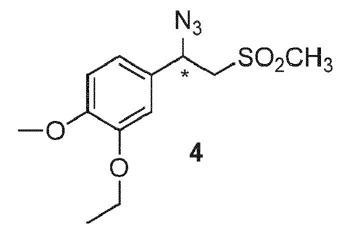
(d) reducing the compound of formula 4 to obtain the compound of formula **5** 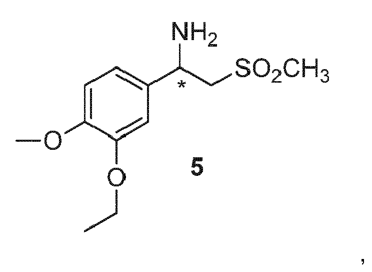 and
(e) reacting the compound of formula **5** with 3-acetamidophthalic anhydride of formula 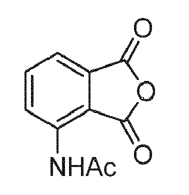 to obtain the compound of formula **6** or a pharmaceutically acceptable salt or solvate thereof.

2. A process for asymmetrically synthesizing a compound of formula **6** or a pharmaceutically acceptable salt or solvate thereof 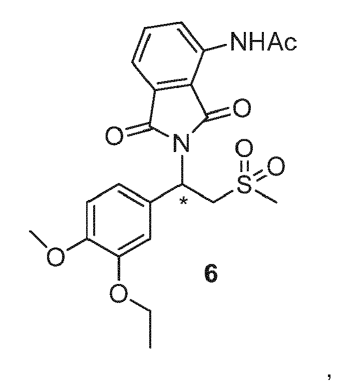 wherein * in the formulae denotes an asymmetric carbon atom in (*R*) or (*S*) configuration being enantiomerically enriched or essentially enantiopure, the process comprising the steps:
(a) reducing a compound of formula **2** 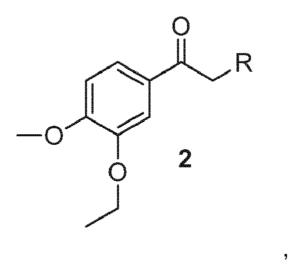 wherein R is methylsulfonyl -SO₂CH₃ or a group convertible to methylsulfonyl,
by an asymmetric enzymatic reduction using baker's yeast containing fungi of the species *Saccharomyces cerevisiae* to give the compound of formula **3** 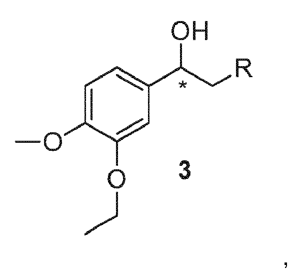 wherein the asymmetric enzymatic reduction leads to the (*R*) or the (*S*) configuration being enantiomerically enriched or essentially enantiopure,
(b) if R is a group convertible to methylsulfonyl converting said group to methylsulfonyl -SO₂CH₃ group,
(c) reacting the compound of formula **3b** 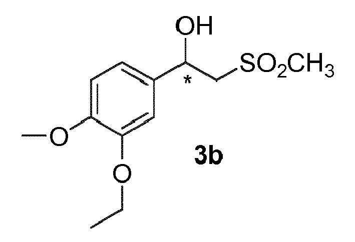 with 3-acetamidophthalimide of formula 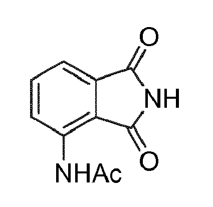 in the presence of a reagent selected from the group consisting of DEAD, DIAD and DCAD, in combination with Ph₃P and Bu₃P, to obtain the compound of formula **6** or a pharmaceutically acceptable salt or solvate thereof.

3. The process according to claim 1 or 2, wherein the compound of formula 3 is produced with the asymmetric carbon atom indicated with * being enantiomerically enriched in the (*R*) configuration, preferably with an ee of at least 60 % ee.

4. The process according to claim 3, wherein the compound of formula **3** with the asymmetric carbon atom indicated with * in the (*R*) configuration, is essentially enantiopure with an ee of at least 75 % ee.

5. The process according to claim 4, wherein the compound of formula **3** with the asymmetric carbon atom indicated with * in the (*R*) configuration, is essentially enantiopure with an ee of at least 85 % ee.

6. The process according to claim 1 or 2, wherein the baker's yeast containing fungi of the species *Saccharomyces cerevisiae* is in a water medium, optionally comprising glucose.

7. The process according to claim 1 or 2, wherein the group convertible to methylsulfonyl is selected from the group consisting of methylsulfenyl, methylsulfinyl, -F, -Cl, -Br and -I.

8. The process according to claim 7, wherein the group convertible to methylsulfonyl is selected from the group consisting of -F, -Cl, -Br and -I.

9. The process according to claim 8, wherein the group convertible to methylsulfonyl is -Cl.

10. A compound of formula **2a**

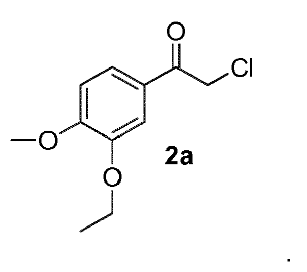

11. A compound of formula **3a** 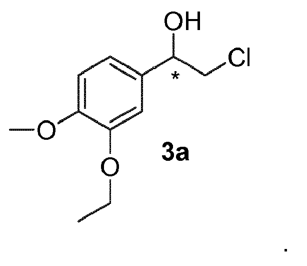 wherein * in the formulae denotes an asymmetric carbon atom in (*R*) or (*S*) configuration being enantiomerically enriched.

12. A compound of the formula **3a*R***

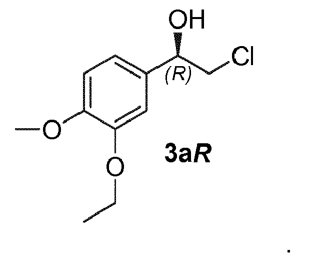

13. Use of the compound as defined in any of the claims 10 to 12 in the preparation of *N*-{2-[(1*S*)-1-(3-ethoxy-4-methoxyphenyl)-2-(methylsulfonyl)ethyl]-1,3-dioxo-2,3-dihydro-1*H-*isoindol-4-yl}acetamide or a pharmaceutically acceptable salt or solvate thereof.

14. A process for the preparation of a pharmaceutical composition comprising *N*-{2-[(1*S*)-1-(3-ethoxy-4-methoxyphenyl)-2-(methylsulfonyl)ethyl]-1,3-dioxo-2,3-dihydro-1*H*-isoindol-4-yl}acetamide or a pharmaceutically acceptable salt or solvate thereof, comprising the steps of:
x) preparing *N*-{2-[(1*S*)-1-(3-ethoxy-4-methoxyphenyl)-2-(methylsulfonyl)ethyl]-1,3-dioxo-2,3-dihydro-1*H*-isoindol-4-yl}acetamide or a pharmaceutically acceptable salt or solvate thereof by carrying out the process according to any of claims 3 to 5, and
y) mixing said obtained *N*-{2-[(1*S*)-1-(3-ethoxy-4-methoxyphenyl)-2-(methyl-sulfonyl)ethyl]-1,3-dioxo-2,3-dihydro-1*H*-isoindol-4-yl}acetamide or a pharmaceutically acceptable salt or solvate thereof with a pharmaceutically acceptable carrier and/or excipient.
